# EUROPEAN PATENT APPLICATION

(11) **EP 2 407 239 A1**
(43) Date of publication of application: **18.01.2012**
(21) Application number: 11173952.0
(22) Date of filing: 14.07.2011
(51) Int. Cl.: B01J 29/04, C07B 61/00, C07C 249/04

(54) **Method for producing oxime**

(30) Priority: 15.07.2010 JP 2010160302
(71) Applicant: Sumitomo Chemical Company, Limited, Tokyo 104-8260 (JP)
(72) Inventor: Tsujiuchi, Sho, Niihama-shi, Ehime (JP)
(74) Representative: Vossius & Partner

(57) **Abstract**

The present invention provides a method for producing an oxime comprising the step of an ammoximation reaction of a ketone with an organic peroxide and ammonia in the presence of a catalyst containing titanium and silicon oxide, wherein the catalyst containing titanium and a silicon oxide is a mesoporous silicate, and is subjected to a contact treatment with a silicon compound.

## Description

### Background of the Invention

### 1. Field of the Invention

The present application claims the Paris Convention priority based on Japanese Patent Application No. 2010-160302 filed on July 15, 2010, the entire content of which is incorporated herein by reference.

The present invention relates to a method for producing an oxime by an ammoximation reaction of a ketone. The oxime is useful as a starting material for an amide or a lactam.

### 2. Description of the Related Art

As a method for producing an oxime by an ammoximation reaction of a ketone, JP-A-2006-169168, JP-A-2007-1952, JP-A-2007-238541 and JP-A-2010-24144 disclose methods in which a ketone is subjected to an ammoximation reaction with an organic peroxide and ammonia in the presence of a catalyst containing titanium and silicon oxide.

### Summary of the Invention

The methods described above, however, are not necessarily sufficient in terms of the yield of an oxime. An object of the present invention is to provide an improved method for producing an oxime by an ammoximation reaction of a ketone with an organic peroxide and ammonia.

That is, the present invention provides a method for producing an oxime comprising the step of an ammoximation reaction of a ketone with an organic peroxide and ammonia in the presence of a catalyst containing titanium and silicon oxide, wherein the catalyst is a mesoporous silicate, and is subjected to a contact treatment with a silicon compound.

According to the present invention, an oxime can be produced in a good yield by an ammoximation reaction of a ketone with an organic peroxide and ammonia.

The present invention encompasses the following embodiments:
(1) A method for producing an oxime comprising the step of an ammoximation reaction of a ketone with an organic peroxide and ammonia in the presence of a catalyst containing titanium and silicon oxide, wherein the catalyst is a mesoporous silicate, and is subjected to a contact treatment with a silicon compound.
(2) The production method according to the item (1), wherein the organic peroxide is hydroperoxide.
(3) The production method according to the item (1) or (2), wherein the mesoporous silicate is HMS or MCM-41.
(4) The production method according to any one of the items (1) to (3), wherein the silicon compound is at least one compound selected from the group consisting of an alkoxysilane compound, an organic disilazane compound and a halogenated organic silane compound.
(5) The production method according to any one of the items (1) to (4), wherein the silicon compound is at least one compound selected from the group consisting of trialkylalkoxysilanes and hexaalkyldisilazanes.
(6) The production method according to any one of the items (1) to (5), wherein the ammoximation reaction is performed by supplying the ketone and ammonia to a reactor in which a solvent, the catalyst and the peroxide are put in advance.
(7) The production method according to any one of the items (1) to (6), wherein the ammoximation reaction is performed by supplying the ketone, the peroxide and ammonia to a reactor in which a solvent, the catalyst, the peroxide and ammonia are put in advance.
(8) The production method according to any one of the items (1) to (7), wherein the ketone is a cycloalkanone.
(9) A process for producing an oxime, which comprises the steps of bringing a titanium containing mesoporous silicate into contact with an organic silicon compound, and then reacting ketone, such as cycloalkanone with ammonia and an organic peroxide in the presence of the resulting titanium containing mesoporous silicate modified with the organic silicon compound.

### Detailed Description of the Invention

Hereinafter, the present invention will be described in detail.

In the present invention the following preferred definitions apply:
"Alkyl" preferably refers to an alkyl group having 1 to 6 carbon atoms.
"Alkenyl" preferably refers to an alkenyl group having 1 to 6 carbon atoms.
"Alkoxy" preferably refers to an alkoxy group having 1 to 6 carbon atoms.
"Aryl" preferably refers to an aryl group having 5 to 10 carbon atoms.
"Cycloalkanone" preferably refers to a cycloalkanone group having 5 to 14 carbon atoms.
"Cycloallcenone" preferably refers to a cycloalkenone group having 5 to 14 carbon atoms." "

The ketone as a starting material may be an aliphathic ketone, an alicyclic ketone, an aromatic ketone, or a combination of two or more kinds thereof. Examples of the ketone include dialkyl ketones, such as acetone, ethyl methyl ketone, and isobutyl methyl ketone; alkyl alkenyl ketones such as mesityl oxide; alkyl aryl ketones, such as acetophenone; diaryl ketones such as benzophenone; cycloalkanones, such as cyclopentanone, cyclohexanone, cyclooctanone, and cyclododecanone; and cycloalkenones such as cyclopentenone and cyclohexenone. Among them, cycloalkanones are suitable.

The ketone as a starting material may be, for example, one obtained by oxidation of an alkane, one obtained by oxidation (dehydrogenation) of a secondary alcohol, or one obtained by hydration and oxidation (dehydrogenation) of an alkene.

Ammonia may be used in a gaseous state or a liquid state, or may be used as a solution thereof in an organic solvent. When gaseous ammonia is used, it is diluted with an inert gas, if necessary. Example of the inert gas may include nitrogen, carbon dioxide, helium, and argon. The amount of ammonia used is preferably adjusted so as to be an ammonia concentration of 1% by weight or more in the liquid phase of a reaction mixture. As seen above, when the ammonia concentration is adjusted to a specified value or more in the liquid phase of a reaction mixture, the conversion ratio of the ketone as a starting material and the selectivity of the oxime as the desired product can be increased; as a result, the yield of the oxime as the desired product can be increased. The ammonia concentration is preferably 1.5% by weight or more, and it is usually 10% by weight or less, preferably 5% by weight or less. The amount of ammonia that may be used is typically 1 mole or more, especially 1.5 moles or more per mole of the ketone.

In the ammoximation reaction of the present invention, a solvent is usually used. Examples of the solvent include hydrocarbons, such as butane, pentane, hexane, cyclohexane, benzene, cumene, toluene, and xylene; nitriles, such as acetonitrile, propionitrile, butyronitrile, isobutyronitrile, trimethyl acetonitrile, valeronitrile, isovaleronitrile, and benzonitrile; and alcohols, such as methyl alcohol, ethyl alcohol, n-propyl alcohol, isopropyl alcohol, n-butyl alcohol, s-butyl alcohol, t-butyl alcohol, and t-amyl alcohol. Among them, hydrocarbons and nitrites are particularly suitable. A combination of two or more kinds thereof may also be used.

When the solvent is used, the amount thereof is usually from 1 to 500 parts by weight, preferably from 2 to 300 parts by weight, based on 1 part by weight of the ketone.

The ammoximation reaction is performed in the presence of a catalyst containing titanium and silicon oxide, which is a mesoporous silicate, and is subjected to a contact treatment with a silicon compound.

Examples of the mesoporous silicate include, for example, MCM-41, MCM-48, HMS, SBA-15, FSM-16, MSU-H, and MSU-F. Among these, titanium-containing MCM-41 and titanium-containing HMS are preferable (hereinafter, titanium-containing MCM-41 and titanium-containing HMS may be, respectively, referred to as "Ti-MCM-41" and "Ti-HMS"). It is noted that "mesoporous silicate" refers to a mesoporous silicate having a pore size of about 2 to about 50 nm. Whether there is a mesoporous structure or not can be confirmed by the presence or absence of a peak at 2θ = 0.2 to 4.0° in an XRD (X-ray diffraction) measurement using Cu K-α radiation. Titanium in the titanium-containing mesoporous silicate may be titanium that has been incorporated into the framework of the silicate or into pores, or carried on the surface of the framework of the silicate. The titanium-containing mesoporous silicate, the titanium-containing MCM-41, and the titanium-containing HMS preferably contain titanium in the framework of the silicate.

The mesoporous silicate containing titanium and silicon oxide may contain boron, aluminum, gallium, iron, chromium and the like in addition to titanium, silicon and oxygen. The elements other than the titanium, silicon and oxygen may be incorporated into the framework of the silicate or into pores, or may be carried on the surface of the framework of the silicate. The mesoporous silicate may contain titanium in the framework of the silicate (titanosilicate), and typically comprises titanium, silicon and oxygen as elements forming the framework, and the framework may be substantially formed from titanium, silicon and oxygen alone, or may contain elements other than titanium, silicon and oxygen, such as boron, aluminum, gallium, iron and chromium, as the elements forming the framework.

The content of titanium in the mesoporous silicate containing titanium and a silicon oxide described above is usually 0.0001 or more, preferably 0.005 or more, and it is usually 1.0 or less, preferably 0.5 or less, in an atomic ratio thereof to silicon (Ti/Si). It is noted that, in a case where the catalyst containing titanium and silicon oxide contains elements other than titanium, silicon and oxygen, the content of the elements is usually 1.0 or less, preferably 0.5 or less, in an atomic ratio thereof to silicon. Oxygen is also contained depending on the content and the oxidation number of each element other than oxygen. The typical composition of the catalyst containing titanium and silicon oxide can be shown by the following formula, using silicon as a basis (=1).

SiO₂·xTiO₂·yMⁿO_{n/2}

wherein M is at least one element other than silicon, titanium and oxygen; n is the oxidation number of the element; x is from 0.0001 to 1.0; and y is from 0 to 1.0.

It is noted that, in the formula described above, M is an element other than titanium, silicon and oxygen, and includes, for example, boron, aluminum, gallium, iron, and chromium.

The mesoporous silicate containing titanium and a silicon oxide can be prepared by a hydrothermal synthesis method, a sol-gel method, or the like. For example, the titanium-containing mesoporous silicate is typically prepared as follows. After a titanium compound as a starting material, a silicon compound as a starting material, and a structure-directing agent (template) are mixed in an aqueous solvent in the presence of an acidic compound or a basic compound, the resulting mixture is aged under constant temperature and pressure conditions or under variable temperature and/or pressure conditions within a range of temperatures and pressures described below to obtain a titanium-containing silicate into which the structure-directing agent is incorporated, and the structure-directing agent is removed from this titanium-containing silicate to prepare a titanium-containing mesoporous silicate.

The structure of the titanium-containing mesoporous silicate can be controlled by the kind, the amount or the like of the structure-directing agent used. For example, when Ti-MCM-41 is prepared, a quaternary ammonium salt such as cetyltrimethylammonium bromide is used, and when Ti-HMS is prepared, a primary amine such as n⁻dodecylamine is used. On the other hand, examples of the titanium compound as a starting material include tetraalkyl orthotitanates such as tetra-n-butyl orthotitanate; peroxytitanates such as tetra-n-butylammonium peroxytitanate; and titanium halides, and examples of the silicon compound as a starting material include tetraalkyl orthosilicates such as tetraethyl orthosilicate, and silicas. Examples of the acidic compound include inorganic acids such as hydrogen chloride; and organic acids such as acetic acid, and examples of the basic compound include inorganic bases such as alkali hydroxides and ammonia; and organic bases such as pyridine. Examples of the aqueous solvent include water, water-soluble organic solvents such as methanol, ethanol, propanol and 2-propanol, and mixed solvents of water and the water-soluble organic solvent.

When the titanium-containing mesoporous silicate is prepared, it is aged at a temperature of, usually, -20 to 200°C, and preferably 20 to 170°C under an absolute pressure of, usually, 0.1 to 1.0 MPa, and preferably 0.1 to 0.8 MPa. The aging time is usually from 0.5 to 170 hours, and preferably from 4 to 72 hours.

When the titanium-containing mesoporous silicate is prepared, the titanium-containing silicate into which the structure-directing agent is incorporated can be obtained by the aging described above, and then the structure-directing agent is removed from this titanium-containing silicate. Examples of the removal method may include a washing method with an organic solvent such as methanol, acetone or toluene; a washing method with hydrochloric acid (aqueous solution of hydrogen chloride), an aqueous sulfuric acid solution, an aqueous nitric acid solution or the like; and a heat-treatment method at 200 to 800°C. The removal methods may be employed alone or as a combination of two or more.

It is noted that Ti-MCM-41 can be prepared in accordance with, for example, a method described in "Microporous and Mesoporous Materials", 2007, pp 312-321; and Ti-HMS can be prepared in accordance with, for example, a method described in "Nature", 1994, pp 321-323.

The mesoporous silicate containing titanium and silicon oxide is subjected to a contact treatment with a silicon compound. The contact treatment is typically conducted prior to the ammoximation reaction. In some embodiments, the resulting mesoporous silicate modified with silicon compound is isolated and then added to the ammoximation reaction. Examples of the silicon compound include organic silicon compounds and inorganic silicon compounds, and organic silicon compounds are preferable among them. Organic silicon compounds capable of bonding to the surface of the mesoporous silicate containing titanium and a silicon oxide by reaction therewith are preferred. Among them, alkoxysilane compounds, organic disilazane compounds and halogenated organic silane compounds are preferable, and alkoxysilane compounds and organic disilazane compounds are more preferable. The alkoxysilane compound, the organic disilazane compound, and the halogenated organic silane compound may be used alone or as a mixture of the two or more kinds thereof. Examples of the alkoxysilane compound include tetraalkoxysilanes, alkyltrialkoxysilanes, dialkyldialkoxysilanes, and trialkylalkoxysilanes, and trialkylalkoxysilanes are preferable among them. Examples of the tetraalkoxysilane include tetramethyl orthosilicate, tetraethyl orthosilicate, tetrapropyl orthosilicate, and tetrabutyl orthosilicate; examples of the alkyltrialkoxysilane include methyltrimethoxysilane and methyltriethoxysilane; examples of the dialkyldialkoxysilane include dimethyldiethoxysilane; and examples of the trialkylalkoxysilane include trimethylethoxysilane and trimethylmethoxysilane. Examples of the organic disilazane compound include hexaalkyldisilazanes such as hexamethyldisilazane and di-n-butyltetramethyldisilazane, divinyltetramethyldisilazane, diphenyltetramethyldisilazane, and tetraphenyldimethyldisilazane, and hexaalkyldisilazanes are preferable among them. Examples of the halogenated organic silane compound include halogenated alkylsilanes such as chlorotrimethylsilane, dichlorodimethylsilane, trichloromethylsilane, chlorobromodimethylsilane, and iododimethylbutylsilane. It is preferable to use at least one compound selected from the group consisting of trialkylalkoxysilanes and hexaalkyldisilazanes as the organic silicon compound, because a hydroxyl group on the surface of the catalyst can be converted into a trialkylsilyl group.

Examples of the inorganic silicon compound include silicic acid, silica gel, fumed silica, and colloidal silica.

Examples of the contact treatment method with the silicon compound include a method in which the mesoporous silicate containing titanium and silicon oxide is immersed in a liquid or a slurry containing the silicon compound; and a method in which a gas containing the silicon compound is brought into contact with the mesoporous silicate containing titanium and silicon oxide. The contact treatment can be performed under an acidic condition, a basic condition or a neutral condition while an acid or a base is appropriately added, or it may be performed while the acidic, basic or neutral condition is changed into another condition during the contact treatment. It is preferable to perform the immersing described above while stirring. According to the immersing described above, the catalyst used for the ammoximation reaction, which is the mesoporous silicate containing titanium and silicon oxide, and is subjected to the contact treatment with the silicon compound, can be obtained by, for example, drying the mixture as it is after immersing it, or by separating the resulting catalyst containing titanium and silicon oxide from the mixture after the immersing through filtration, decantation or the like, then washing it if necessary, and drying it. The drying can be performed either under an ordinary pressure or under a reduced pressure, the drying temperature is preferably from 20 to 150°C, and the drying time is preferably from 0.5 to 100 hours.

The amount of the silicon compound that may be used is usually from 1 to 10000 parts by weight, preferably from 5 to 2000 parts by weight, and more preferably from 10 to 1500 parts by weight, based on 100 parts by weight of the catalyst containing titanium and a silicon oxide. It is noted that when two or more compounds selected from the group consisting of an alkoxysilane compound, an organic disilazane compound and a halogenated organic silane compound are used as silicon compounds, as described above, the amount may be adjusted so that the total used amount is within the range described above. Also, when the trialkylalkoxysilane and the hexaalkyldisilazane are used as silicon compounds, as described above, the amount may be adjusted so that the total used amount is within the range described above.

The contact treatment with the silicon compound is performed preferably at a temperature of 0 to 200°C, and more preferably 30 to 100°C when the catalyst is immersed in the liquid or slurry, and preferably at a temperature of 0 to 800°C, and more preferably 100 to 500°C when the catalyst is brought into contact with the gas containing the silicon compound. The contact treatment time is preferably from 0.5 to 50 hours, and more preferably from 1 to 20 hours when the catalyst is immersed in the liquid or slurry, and it is from 0.5 to 100 hours, and preferably from 1 to 50 hours when the catalyst is brought into contact with the gas containing the silicon compound.

In the preparation of the liquid or slurry containing the silicon compound, a solvent may be used in order to make the silicon compound stable. Also, the liquid containing the silicon compound and a solvent is evaporated, and the resulting gas may be used as the gas containing the silicon compound. Examples of the solvent may include water, methanol, ethanol, acetonitrile, toluene, xylene, cumene, tetrahydrofuran, carbon tetrachloride, and N,N-dimethyl acetamide. These solvents may be used alone or as a mixture of two or more thereof.

The treatment in which the catalyst is brought into contact with the gas containing the silicon compound may be performed in the presence of an inert gas together with the gas containing the silicon compound. Examples of the inert gas include nitrogen, carbon dioxide, helium, and argon.

The mesoporous silicate containing titanium and a silicon oxide may be molded into particles, pellets or the like using a binder if necessary, and the resulting molded articles may be used, or it may be supported on a carrier and the resulting supported product may be used. The molding treatment or carrying treatment may be performed either before or after the contact treatment with the silicon compound.

The organic peroxide is converted into an alcohol or a carboxylic acid, but they can be recovered by distillation or extraction, which is advantageous in terms of the cost for the raw materials and effective use. For example, when cumene hydroperoxide is used as an organic peroxide, 2-phenyl-2-propanol, obtained after the ammoximation reaction, can be recovered as cumene hydroperoxide by hydrogenation and oxidation, and it can be reused.

Examples of the organic peroxide referred to herein include hydroperoxides such as t-butyl hydroperoxide, cumene hydroperoxide, cyclohexyl hydroperoxide, diisopropylbenzene hydroperoxide, p-menthane hydroperoxide, and 1,1,3,3-tetramethylbutyl hydroperoxide; dialkyl peroxides such as t-butyleumyl peroxide, di-t-butyl peroxide, di-t-hexyl peroxide, dicumyl peroxide, α,α'-di(t-butylperoxy)diisopropylbenzene, 2,5-dimethyl-2,5-di(t-butylperoxy)hexane, and 2,5-dimethyl-2,5-bis(t-butylperoxy)hexyne-3; peroxy esters such as cumyl peroxyneodecanoate, 1,1,3,3-tetramethylbutyl peroxyneodecanoate, t-hexyl peroxyneodecanoate, t-butyl peroxyneodecanoate, t-butyl peroxyneoheptanoate, t-hexyl peroxyvalerate, t-butyl peroxypivalate, 2,5-dimethyl-2,5-di(2-ethylhexanoylperoxy)hexane, 1,1,3,3-tetramethylbutyl peroxy-2-ethylhexanoate, t-hexylperoxy-2-ethyl hexanoate, t-butylperoxy-2-ethyl hexanoate, t-butylperoxylaurate, t-butylperoxy-3,5,5-trimethyl hexanoate, t-hexylperoxyisopropyl monocarbonate, t-butyl-peroxy-2-ethylhexyl monocarbonate, 2,5-dimethyl-2,5-di(benzoylperoxy)hexane, t-butyl peroxyacetate, t-hexyl peroxybenzoate, and t-butylperoxybenzoate; diacyl peroxides such as diisobutyryl peroxide, di(3,5,5-trimethylhexanoyl)peroxide, dilauroyl peroxide, disuccinic acid peroxide, dibenzoyl peroxide, and di(4-methylbenzoyl)peroxide; and peroxydicarbonates such as diisopropyl peroxydicarbonate, di-n-propyl peroxydicarbonate, bis(4-t-butylcyclohexyl)peroxydicarbonate, di-2-ethylhexyl peroxydicarbonate, and di-sec-butyl peroxydicarbonate. Among them, hydroperoxide is preferable.

The ammoximation reaction may be performed in a batch mode, a semi-batch mode, a continuous mode, or a combination of the batch, semi-batch and/or continuous modes. The semi-batch mode, the continuous mode and the combination thereof are preferable among them. In a case of the semi-batch mode, it is preferable to perform the reaction while reaction starting materials are fed into a stirring and mixing type reactor or a loop reactor. In a case of the continuous mode, it is desirable to perform the reaction in a mode in which a liquid phase of a reaction mixture is withdrawn while reaction starting materials are fed into a stirring and mixing type reactor or a loop reactor, or a fixed-bed flow mode in which reaction starting materials are fed into a fixed-bed reactor packed with the catalyst, in terms of the productivity and the operability.

The semi-batch mode reaction using the stirring and mixing type reactor can be preferably performed by, for example, feeding reaction starting materials such as a ketone to the reactor so that a reaction mixture in which the catalyst containing titanium and a silicon oxide is suspended exists in the realtor. The continuous mode reaction using the stirring and mixing type reactor can be suitably performed by, for example, feeding reaction starting materials such as a ketone into the reactor so that a reaction mixture in which the catalyst containing titanium and a silicon oxide is suspended exists in the reactor, and at the same time, withdrawing the liquid phase of the reaction mixture from the reactor through a filter.

In the semi-batch mode or continuous mode reaction using the stirring and mixing type reactor, it is preferable to feed a ketone and ammonia into the reactor in which the solvent, the catalyst and the peroxide are put in advance, and it is more preferable to feed a ketone, the peroxide and ammonia into the reactor in which the solvent, the catalyst, the peroxide and ammonia are put in advance. Specifically, the solvent, the catalyst and the peroxide are first introduced into the stirring and mixing type reactor. The introduction order thereof is not particularly limited. After the introduction thereof into the reactor, the mixture is stirred to suspend the catalyst therein. Next, a ketone and ammonia are fed thereinto. A ketone and ammonia may be fed separately (generally called "co-feed), or the mixture thereof may be fed. Also, ammonia is put in advance in the reactor together with the solvent, the catalyst and the peroxide, and then a ketone and additional ammonia may be fed into the reactor; the solvent, the catalyst and the peroxide are put in advance in a reactor, and then the additional peroxide may be fed into the reactor together with a ketone and ammonia; and ammonia is put in advance in the reactor together with the solvent, the catalyst and the peroxide, and then the additional peroxide is fed into the reactor together with a ketone and the additional ammonia. It is noted that the ketone, the ammonia and the peroxide used for feeding may be diluted with a solvent.

The amount of the catalyst that may be used in the semi-batch mode or continuous mode reaction using the stirring and mixing type reactor may be from about 0.1 to 20% by weight based on the total amount of the reaction mixture. For the purpose of suppressing the reduction in catalyst activity, as shown in, for example, JP-A-2004-83560, a silicon compound such as silica or silicic acid may co-exist in the reaction system.

In the semi-batch mode or continuous mode reaction using the stirring and mixing type reactor, when an organic peroxide is put in advance in the reactor, the amount of the organic peroxide which is put in advance is adjusted so that a concentration of the organic peroxide in the liquid phase of the mixture in the reactor is from 0.01 to 50% by weight. Also, in the semi-batch mode or continuous mode reaction using the stirring and mixing type reactor, when ammonia is put in advance in the reactor, the amount of the ammonia which is put in advance is adjusted so that a concentration of the ammonia in the liquid phase of the mixture in the reactor is from 0.1 to 15% by weight.

The amount of the organic peroxide fed in the semi-batch mode or continuous mode reaction using the stirring and mixing type reactor is usually from 0.5 to 20 moles, and preferably from 0.5 to 15 moles per mole of the ketone.

The amount of the ammonia fed in the semi-batch mode or continuous mode reaction using the stirring and mixing type reactor is usually 1 mole or more per mole of the ketone.

It is noted that the stirring and mixing type reactor is preferably has a glass-lining or is made of stainless steel, from the viewpoint of prevention of decomposition of the organic peroxide.

The reaction in the fixed-bed flow mode can be performed, for example, by feeding upwardly or downwardly a ketone, a peroxide and ammonia, which are reaction starting materials, if necessary together with a solvent, into a fixed-bed reactor packed with the catalyst containing titanium and a silicon oxide. When gaseous ammonia is used as ammonia, the gaseous ammonia is diluted with an inert gas if necessary, and the feeding direction may be either parallel or counter to a direction of feeding the starting materials other than ammonia. The reaction is preferably performed under pressure. It is possible to adjust the contact time of the catalyst with the reaction starting materials by controlling the pressure conditions.

As the fixed-bed reactor, various flow type fixed-bed reactors having an inlet for feeding starting materials and an outlet for withdrawing a reaction liquid can be used. The number of reaction tubes is not particularly limited, and either a single-tube fixed-bed reactor or a multi-tube fixed-bed reactor can be used. Also, a heat insulating fixed-bed reactor or a heat exchanging fixed-bed reactor can be used. A glass-lined reactor or a stainless steel reactor is preferable from the viewpoint of prevention of the decomposition of the organic peroxide.

The reaction temperature in the ammoximation reaction is usually from 50 to 200°C, and preferably from 80 to 150°C. The reaction pressure is usually from 0.1 to 5.0 MPa, preferably from0.2 to 1.0 MPa in absolute pressure. In order to easily dissolve ammonia in the liquid phase of the reaction mixture, the reaction is preferably performed under pressure, and in such a case, the pressure may be adjusted by using an inert gas such as nitrogen or helium.

The after-treatment of the obtained reaction mixture is appropriately selected. For example, the oxime can be separated by removing the catalyst from the reaction mixture by filtration, decantation, or the like, and then subjecting the liquid phase to distillation. The separated catalyst is subjected to treatments such as washing, sintering, and re-contact with a silicon compound if necessary, and the resulting catalyst can be reused. When a solvent and unreacted starting materials are contained in the reaction mixture, the solvent and unreacted starting materials which are recovered by distillation of the liquid phase can be reused. The obtained oxime is suitably used as a starting material for producing an amide compound corresponding thereto by Beckmann rearrangement reaction.

Hereinafter, the Examples and the Comparative Examples of the present invention will be shown, but the present invention is not limited thereto. It is noted that in the Examples and the Comparative Examples, a liquid phase of a reaction mixture was analyzed by gas chromatography, and the conversion ratio of cyclohexanone, and the selectivity and the yield of cyclohexanone oxime were calculated.

### Reference Example 1

In a 100 ml recovery flask were put 102 g of toluene, 2.5 g of Ti-MCM-41, and 7.8 g of hexamethyldisilazane, and the mixture was stirred at room temperature for 5 minutes. Then, the temperature of the mixture was raised in an oil bath while stirring, and after the temperature reached 80°C, the mixture was kept at that temperature for 2 hours. After the mixture was cooled to room temperature, the stirring was stopped, and the mixture was allowed to stand. Then, a supernatant liquid was removed through decantation. The remaining mixture was dried at 60°C for 2 hours under reduced pressure conditions to prepare catalyst A.

### Reference Example 2

Catalyst B was prepared in the same manner as in Reference Example 1, except that Ti-HMS was used instead of Ti-MCM-41.

### Example 1

In a 1-liter autoclave (stirring and mixing type reactor) were put 150.8 g of an acetonitrile solution containing 4.3% by weight of ammonia, 7.7 g of a cumene solution containing 80% by weight of cumene hydroperoxide, and 2.5 g of catalyst A, and a gas phase portion in the reactor was substituted by nitrogen. After that, the reactor was sealed, and the temperature inside the reactor was raised to 120°C while stirring. The pressure inside the reactor was 0.5 MPa at this time. Subsequently, an acetonitrile solution containing 4.7% by weight of cyclohexanone, and an acetonitrile solution containing 2.6% by weight of cumene hydroperoxide and 3.9% by weight of ammonia were continuously separately fed (co-fed) into the reactor in flow rates of 10 g/hour and 115 g/hour, respectively, to start the reaction. The reaction was continued while the liquid phase of the reaction mixture was withdrawn through a sintered metal filter made of stainless steel so that the volume of the reaction mixture in the reactor could be about 250 g at the time when 1 hour passed after starting the reaction. The concentration of ammonia in the liquid phase of the reaction mixture changed within a range of 1.1 to 4.3% by weight of the liquid phase.

The liquid phase of the reaction mixture which was withdrawn at the time when 1 hour passed after starting the reaction was analyzed by gas chromatography, and it was found that the conversion ratio of cyclohexanone was 99.3%, and the selectivity and the yield of cyclohexanone oxime were 91.7% and 91.0%. Also, the production rate of cyclohexanone imine (compound obtained by imination of cyclohexanone) and impurities derived from the imine to the fed cyclohexanone was 8.3%. The liquid phase of the reaction mixture withdrawn at the time when 6 hours passed after starting the reaction was analyzed by gas chromatography, and it was found that the conversion ratio of cyclohexanone was 97.1%, and the selectivity and the yield of cyclohexanone oxime were 81.4% and 79.0%. Also, the production rate of cyclohexanone imine (compound obtained by imination of cyclohexanone) and impurities derived from the imine to the fed cyclohexanone was 16.1%.

### Example 2

In a 1-liter autoclave (stirring and mixing type reactor) were put 153.3 g of an acetonitrile solution containing 3.9% by weight of ammonia, 7.6 g of a cumene solution containing 80% by weight of cumene hydroperoxide, and 2.4 g of catalyst B, and a gas phase portion in the reactor was substituted by nitrogen. After that, the reactor was sealed, and the temperature inside the reactor was raised to 120°C while stirring. The pressure inside the reactor was 0.5 MPa at this time. Subsequently, an acetonitrile solution containing 4.7% by weight of cyclohexanone, and an acetonitrile solution containing 2.6% by weight of cumene hydroperoxide and 3.8% by weight of ammonia were continuously separately fed (co-fed) into the reactor in flow rates of 10 g/hour and 115 g/hour, respectively, to start the reaction. The reaction was continued while the liquid phase of the reaction mixture was withdrawn through a sintered metal filter made of stainless steel so that the volume of the reaction mixture in the reactor could be about 250 g at the time when 1 hour passed after starting the reaction. The concentration of ammonia in the liquid phase of the reaction mixture changed within a range of 1.4 to 3.9% by weight of the liquid phase.

The liquid phase of the reaction mixture which was withdrawn at the time when 1 hour passed after starting the reaction was analyzed by gas chromatography, and it was found that the conversion ratio of cyclohexanone was 99.2%, and the selectivity and the yield of cyclohexanone oxime were 95.5% and 94.7%. Also, the production rate of cyclohexanone imine (compound obtained by imination of cyclohexanone) and impurities derived from the imine to the fed cyclohexanone was 4.5%. The liquid phase of the reaction mixture which was withdrawn at the time when 6 hours passed after starting the reaction was analyzed by gas chromatography, and it was found that the conversion ratio of cyclohexanone was 91.5%, and the selectivity and the yield of cyclohexanone oxime were 98.8% and 90.3%. Also, the production rate of cyclohexanone imine (compound obtained by imination of cyclohexanone) and impurities derived from the imine to the fed cyclohexanone was 1.2%.

### Comparative Example 1

In a 1-liter autoclave (stirring and mixing type reactor) were put 154.6 g of an acetonitrile solution containing 3.0% by weight of ammonia, 7.6 g of a cumene solution containing 80% by weight of cumene hydroperoxide, and 2.5 g of Ti-MCM-41, and a gas phase portion in the reactor was substituted by nitrogen. After that, the reactor was sealed, and the temperature inside the reactor was raised to 120°C while stirring. The pressure inside the reactor was 0.5 MPa at this time. Subsequently, an acetonitrile solution containing 4.7% by weight of cyclohexanone, and an acetonitrile solution containing 2.6% by weight of cumene hydroperoxide and 3.8% by weight of ammonia were continuously separately fed (co-fed) into the reactor in flow rates of 10 g/hour and 115 g/hour, respectively, to start the reaction. The reaction was continued while the liquid phase of the reaction mixture was withdrawn through a sintered metal filter made of stainless steel SO that the volume of the reaction mixture in the reactor could be about 250 g at the time when 1 hour passed after starting the reaction. The concentration of ammonia in the liquid phase of the reaction mixture changed within a range of 1.0 to 3.0% by weight of the liquid phase.

The liquid phase of the reaction mixture which was withdrawn at the time when 1 hour passed after starting the reaction was analyzed by gas chromatography, and it was found that the conversion ratio of cyclohexanone was 94.1%, and the selectivity and the yield of cyclohexanone oxime were 74.9% and 70.5%. Also, the production rate of cyclohexanone imine (compound obtained by imination of cyclohexanone) and impurities derived from the imine to the fed cyclohexanone was 23.6%. The liquid phase of the reaction mixture which was withdrawn at the time when 6 hours passed after starting the reaction was analyzed by gas chromatography, and it was found that the conversion ratio of cyclohexanone was 94.1%, and the selectivity and the yield of cyclohexanone oxime were 73.4% and 69.0%. The production rate of cyclohexanone imine (compound obtained by imination of cyclohexanone) and impurities derived from the imine to the fed cyclohexanone was 25.1%.

### Comparative Example 2

In a 1-liter autoclave (stirring and mixing type reactor) were put 155.0 g of an acetonitrile solution containing 3.3% by weight of ammonia, 7.6 g of a cumene solution containing 80% by weight of cumene hydroperoxide, and 2.5 g of Ti-HMS, and a gas phase portion in the reactor was substituted by nitrogen. After that, the reactor was sealed, and the temperature inside the reactor was raised to 120°C while stirring. The pressure inside the reactor was 0.5 MPa at this time. Subsequently, an acetonitrile solution containing 4.7% by weight of cyclohexanone, and an acetonitrile solution containing 2.6% by weight of cumene hydroperoxide and 3.8% by weight of ammonia were continuously separately fed (co-fed) into the reactor in flow rates of 10 g/hour and 115 g/hour, respectively, to start the reaction. The reaction was continued while the liquid phase of the reaction mixture was withdrawn through a sintered metal filter made of stainless steel so that the volume of the reaction mixture in the reactor could be about 250 g at the time when 1 hour passed after starting the reaction. The concentration of ammonia in the liquid phase of the reaction mixture changed within a range of 1.2 to 3.3% by weight of the liquid phase.

The liquid phase of the reaction mixture which was withdrawn at the time when 1 hour passed after starting the reaction was analyzed by gas chromatography, and it was found that the conversion ratio of cyclohexanone was 94.8%, and the selectivity and the yield of cyclohexanone oxime were 98.9% and 93.8%. Also, the production rate of cyclohexanone imine (compound obtained by imination of cyclohexanone) and impurities derived from the imine to the fed cyclohexanone was 1.0%. The liquid phase of the reaction mixture which was withdrawn at the time when 6 hours passed after starting the reaction was analyzed by gas chromatography, and it was found that the conversion ratio of cyclohexanone was 96.7%, the selectivity and the yield of cyclohexanone oxime were 87.2% and 84.4%. The production rate of cyclohexanone imine (compound obtained by imination of cyclohexanone) and impurities derived from the imine to the fed cyclohexanone was 12.3%.

### Comparative Example 3

In a 1-liter autoclave (stirring and mixing type reactor) were put 155.5 g of an acetonitrile solution containing 3.1% by weight of ammonia, 7.6 g of a cumene solution containing 80% by weight of cumene hydroperoxide, and 2.5 g of Ti-MWW (prepared in the same manner as described in "Chemistry Letters, 2000, pp 774-775), and a gas phase portion in the reactor was substituted by nitrogen. After that, the reactor was sealed, and the temperature inside the reactor was raised to 120°C while stirring. The pressure inside the reactor was 0.5 MPa at this time. Subsequently, an acetonitrile solution containing 4.7% by weight of cyclohexanone, and an acetonitrile solution containing 2.6% by weight of cumene hydroperoxide and 3.8% by weight of ammonia were continuously separately fed (co-fed) into the reactor in flow rates of 10 g/hour and 115 g/hour, respectively, to start the reaction. The concentration of ammonia in the liquid phase of the reaction mixture changed within a range of 1.0 to 3.1% by weight of the liquid phase.

The liquid phase of the reaction mixture which was withdrawn at the time when 1 hour passed after starting the reaction was analyzed by gas chromatography, and it was found that the conversion ratio of cyclohexanone was 67.8%, and the selectivity and the yield of cyclohexanone oxime were 89.8% and 60.9%. The production rate of cyclohexanone imine (compound obtained by imination of cyclohexanone) and impurities derived from the imine to the fed cyclohexanone was 6.9%.

## Claims

1. A method for producing an oxime comprising the step of an ammoximation reaction of a ketone with an organic peroxide and ammonia in the presence of a catalyst containing titanium and silicon oxide, wherein the catalyst is a mesoporous silicate, and is subjected to a contact treatment with a silicon compound.

2. The production method according to claim 1, wherein the organic peroxide is a hydroperoxide.

3. The production method according to claim 1, wherein the mesoporous silicate is HMS or MCM-41.

4. The production method according to claim 1, wherein the silicon compound is at least one compound selected from the group consisting of an alkoxysilane compound, an organic disilazane compound and a halogenated organic silane compound.

5. The production method according to claim 1, wherein the silicon compound is at least one compound selected from the group consisting of trialkylalkoxysilane and hexaalkyldisilazane.

6. The production method according to claim 1, wherein the ammoximation reaction is performed by supplying the ketone and ammonia to a reactor in which a solvent, the catalyst and the peroxide are put in advance.

7. The production method according to claim 1, wherein the ammoximation reaction is performed by supplying the ketone, the peroxide and ammonia to a reactor in which a solvent, the catalyst, the peroxide and ammonia are put in advance.

8. The production method according to claim 1, wherein the ketone is a cycloalkanone.
